# EUROPEAN PATENT APPLICATION

(11) **EP 4 574 090 A1**
(43) Date of publication of application: **25.06.2025**
(21) Application number: 23853849.0
(22) Date of filing: 10.03.2023
(51) Int. Cl.: A61F 2/01

(54) **DEVICE FOR INTERCEPTING THROMBI**

(30) Priority: 17.08.2022 CN 202210984948
(71) Applicant: Shangai Kegang Medical Technology Co., Ltd, Shanghai 201613 (CN)
(72) Inventor: ZHANG, Zhichao, Shanghai 201601 (CN); WANG, Wenzhe, Shanghai 201601 (CN); QIAN, Hengyue, Shanghai 201601 (CN); ZHOU, Zhilong, Shanghai 201601 (CN); MA, Changsheng, Shanghai 201601 (CN); DONG, Jianzeng, Shanghai 201601 (CN)
(74) Representative: Cameron Intellectual Property Ltd
(86) International application number: PCT/CN2023/080753
(87) International publication number: WO 2024/036932

(57) **Abstract**

The present disclosure provides an embolic protection device comprising a metal wire or metal ribbon with an elliptical or flat cross-section, pre-formed into a spiral structure wound around its short axis. The formed embolic protection device can be deployed within a tubular object and introduced into the target blood vessel through the tube. The elasticity of the material enables it to revert to a predetermined shape, thereby effectively intercepting emboli. The device is characterized by a smaller curvature, enhanced recovery to the preset shape, and reduced interference with blood flow.

## Description

### Field of this disclosure

The present disclosure relates to a medical device, particularly an embolic protection device.

### Background of the disclosure

Technologies mentioned in CN104736102A and the patent US20080183206A1 by BATISTE, STANLEY, which are the only known technologies currently available that use a single wire to construct a vascular embolic protection device.

The basic principle of CN104736102A involves presetting a shape using a superelastic metal wire, which is delivered into the target blood vessel cavity via a hollow needle. Upon insertion, the wire recovers to its preset shape and stabilizes within the vessel to intercept thrombi. There are two designs in the existing patents and product information:

One design forms a spiral in the blood vessel with the axis of the spiral perpendicular to the direction of blood flow. While a smaller gap in the spiral can intercept emboli, it reduces blood flow. A larger gap allows emboli to pass through, failing to achieve the purpose of interception.

The other design forms a family of spirals in the blood vessel with mutually parallel but distinct axes, all parallel to the direction of blood flow. This design successfully addresses the balance between blood flow and emboli interception capability. However, to achieve this technical effect, it employs two non-coaxial spirals. Coaxial spirals require a finer wire diameter to intercept emboli effectively, but a finer diameter means the wire is more susceptible to deformation by blood flow, losing its blocking capability. Non-coaxial spirals complicate mold design in production and result in lower heat-setting yields.

The patent by Batiste and Stanley uses coaxial spirals, but since its design environment is for veins and the target emboli size is relatively large, it can achieve the desired effect using a finer metal wire and a larger spiral diameter. However, in cases where the blood vessel wall has strong elasticity, this design can lead to deformation of the embolic protection device due to the pulsation of the blood vessel wall, further causing fluctuations in blood flow. These fluctuations can lead to local hemodynamic turbulence, increasing the likelihood of emboli formation.

Therefore, existing technologies need further improvement to reduce interference with blood flow and to intercept smaller emboli. A new intravascular embolic protection device is required that causes less interference with blood flow, can intercept smaller emboli, and has a smaller curvature for easier deployment and shape recovery.

### Summary of the disclosure

The objective of the present disclosure is to provide an embolic protection device that achieves smaller curvature and deflection, easier recovery to the preset shape, and less interference with blood flow.

To this end, the disclosure provides an embolic protection device using a metal wire or metal ribbon with an elliptical or flat cross-section pre-shaped into a spiral form wound around the short axis. The formed embolic protection device can be deployed within a tubular object and introduced into the target blood vessel through the tube. The elasticity of the material allows it to return to the predetermined shape to intercept emboli.

In this embodiment, the cross-section of the metal wire or metal ribbon is elliptical or streamlined. The short axis of the elliptical or streamlined cross-section faces the direction of blood flow and is tangent to it. The long axis of the elliptical or streamlined cross-section is parallel to the direction of blood flow. The length-to-width ratio of the elliptical or streamlined cross-section is greater than 1.5. The ratio of the short axis to the minimum intercept diameter should be less than the maximum elastic strain of the material.

Preferably, the angle between the axis of the spiral and the long axis of the elliptical or streamlined cross-section is an acute angle, preferably less than 30 degrees.

The angle between the axis of the spiral and the wide face of the elliptical or streamlined cross-section is also an acute angle.

The beneficial effects of the present disclosure are as follows:
In this embodiment, the winding around the short axis avoids excessive surface strain and stress in the material, ensuring that the embolic protection device can recover its shape. This method allows for the creation of a more curved interception area, ensuring interception performance. The performance of the embolic protection device is optimized, allowing for a smaller interception diameter. It improves the hemodynamic environment of the embolic protection device within the blood vessel, reduces turbulence, and further decreases the likelihood of emboli formation.

### Brief Description of the drawings

Fig. 1: Schematic diagram of the overall structure of the disclosure before use.
Fig. 2: Structural diagram of one embodiment of the disclosure.
Fig. 3: Cross-sectional view of a single wire of the disclosure.
Fig. 4: Comparative diagram of spirals wound around the short axis and long axis.
Fig. 5: Schematic diagram of one embodiment of the disclosure facing the direction of blood flow.
Fig. 6: Cross-sectional view along line A-A of Fig. 5.
Fig. 7: Enlarged view of the circled part in Fig. 6.

### Detailed Description of some of the embodiments

The present disclosure is primarily an embolic protection device, characterized by using a metal flat wire or metal ribbon with an elliptical, flat, or streamlined cross-section pre-shaped into a spiral or helical form, serving as an intravascular implant for intercepting emboli.

The formed embolic protection device can be deployed within a tubular object and introduced into the target blood vessel through the tube. The elasticity of the material allows it to return to the predetermined shape to intercept emboli. In this embodiment, the elliptical flat wire can be wound around the short axis to avoid excessive surface strain and stress in the material, ensuring that the embolic protection device can recover its shape. This method allows for the creation of a more curved interception area, ensuring interception performance.

In this embodiment, the cross-section of the embolic protection device preferably has an elliptical or streamlined shape, with the short axis of the elliptical or streamlined cross-section facing the direction of blood flow, minimizing blood flow resistance and maintaining laminar flow along the device.

Additionally, the long axis of the cross-section is parallel to the direction of blood flow. The impact force of the blood flow on the short axis is converted into a torsional moment on the long axis, which has a higher flexural rigidity, resulting in minimal deformation of the embolic protection device under impact, maintaining its shape.

The following detailed description refers to the accompanying drawings and specific embodiments to further illustrate the disclosure.

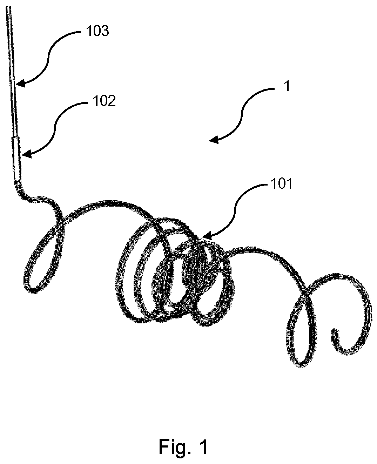

Refer to Fig. 1, which is a schematic diagram of the overall structure of the disclosure before use. As shown in Fig. 1, one embodiment of the embolic protection device of the present disclosure is a curve formed by winding a single wire. The overall structure of the embodiment in Fig. 1 includes three parts: the embolic protection device 101, the connecting ring 102, and the traction wire 103. **In** this embodiment, the embolic protection device 101 is characterized by a spiral line formed from a superelastic metal wire with a flat cross-section, where the angle between the axis of the spiral and the wide face of the flat wire is an acute angle. This design minimizes the strain of the embolic protection device 101, ensuring that the metal wire can recover its original shape after passing through a long straight channel. Additionally, in the axial direction, its thickness is greater than that of the flat wire (usually more than 1.5 times the thickness of the flat wire), which minimizes the deformation of the embolic protection device 101 when subjected to blood flow impact, thereby avoiding disturbances in blood flow caused by deformation. Furthermore, as emboli accumulate on the embolic protection device 101 and are flushed by blood flow, the cross-section of the flat wire provides a significant local stress, which cuts the soft emboli into smaller pieces, preventing blockages caused by large emboli. In this embodiment, the connecting ring 102 is preferably made of a biodegradable metal material, such as magnesium alloy or iron alloy, or a non-biodegradable material with good biocompatibility, such as titanium alloy or stainless steel. Its primary function is to connect the embolic protection device 101 with the traction wire 103. After implantation, the connecting ring 102 remains on the blood vessel wall for a period of time, during which it becomes endothelialized at a rate faster than its degradation rate. In this embodiment, the traction wire 103 is designed to allow immediate removal of the embolic protection device from the blood vessel by pulling the traction wire 103 if the device does not meet expectations or problems arise after implantation. The traction wire 103 is made of an absorbable suture material capable of withstanding tensile forces.

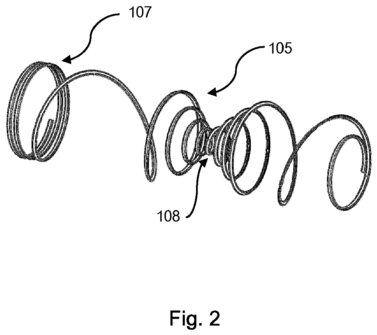

Fig. 2 illustrates the structural diagram of one embodiment of the disclosure. In this embodiment, the embolic protection device 105 is composed of a single superelastic metal wire, characterized by a spiral line formed from a superelastic metal wire with a flat cross-section. The fixing section 107 can secure the embolic protection device to the blood vessel wall by tension. The diameter of the central interception section 108 is smaller than the minimum diameter of the target emboli. Compared to the first embodiment, the advantage here is that no foreign object spans across the blood vessel wall after implantation, although the foreign object in the previous embodiment will eventually degrade and become endothelialized.

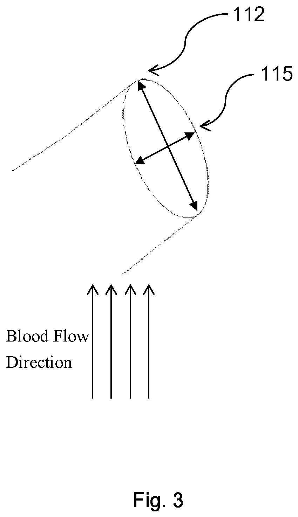

Fig. 3 is a cross-sectional view of a single wire of the disclosure. As shown in Fig. 3, the cross-section of the single wire is approximately elliptical, including a long axis 112 and a short axis 115. In this embodiment, the short axis 115 faces the direction of blood flow and is tangent to it. The ratio of the short axis 115 to the minimum intercept diameter 109 should be less than the maximum elastic strain of the material.

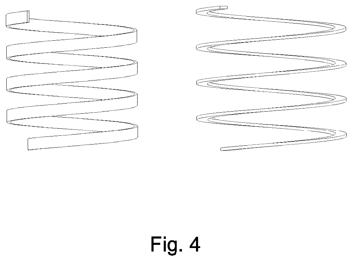

Fig. 4 is a comparative diagram of spirals wound around the short axis and long axis. In this embodiment, an elliptical flat wire can be wound around the short axis to avoid excessive surface strain and stress in the material, ensuring that the embolic protection device can recover its shape. In Fig. 4, the winding method on the left is referred to as winding around the long axis, while the method on the left is referred to as winding around the short axis.

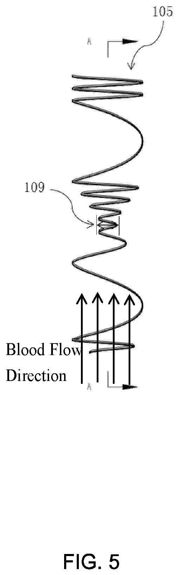

Fig. 5 is a schematic diagram of one embodiment of the disclosure facing the direction of blood flow. Fig. 5 shows the direction of blood flow facing the embolic protection device 105, with the minimum intercept diameter 109 being the smallest diameter of the spiral loop of the embolic protection device 105.

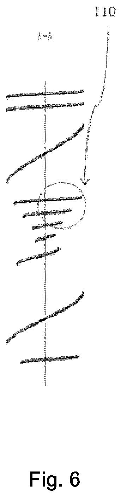

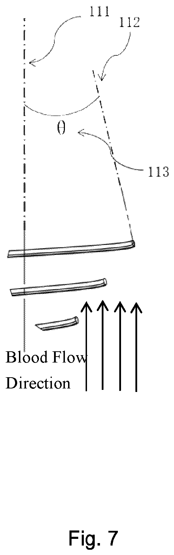

Fig. 6 is a cross-sectional view along line A-A of Fig. 5. Fig. 7 is an enlarged view of the circled part in Fig. 6.

In this embodiment, the cross-section of the single wire is streamlined. Within the circle 110, the angle 113 between the long axis 112 of the single wire's cross-section and the main axis 111 of the embolic protection device's spiral is an acute angle less than 45 degrees, preferably less than 30 degrees. Under these conditions, when blood flow impacts the short axis of the cross-section, the blood flow adheres to the surface of the embolic protection device, reducing the likelihood of local vortices or turbulence. Within the acute angle range, no negative pressure is generated in the local flow field of the blood flow.

Finite element analysis was used to simulate the different cross-sectional designs of the embolic protection device on the pressure on the blood vessel wall and the impact on blood flow. The simulation assumed that the blood vessel wall was in a systolic pressure (high-pressure) state, with an inner diameter 10% smaller than the outer diameter of the embolic protection device. The systolic blood pressure was 120 mmHg, and the diastolic pressure was 80 mmHg. The pressure and velocity changes in the main branch arteries (brachial artery, carotid artery) were used as boundary conditions, yielding the following results:
- For an embolic protection device with a circular cross-section of 0.25 mm in diameter, the maximum instantaneous pressure on the blood vessel wall was 52.3 ± 7.8 kPa.
- The local pressure difference before and after the embolic protection device with a circular cross-section of 0.25 mm in diameter was approximately 7.5 mmHg.
- For an embolic protection device with an elliptical cross-section of 0.25 mm x 0.12 mm, the maximum instantaneous pressure on the blood vessel wall was 27.6 ± 4 kPa.
- The local pressure difference before and after the embolic protection device with an elliptical cross-section was approximately 5.7 mmHg.

Compared to a circular cross-section, the elliptical cross-section exerts less pressure on the blood vessel wall, making it less likely to cause intimal proliferation. Additionally, the blood flow pressure drop across an elliptical cross-section is significantly lower than that across a circular cross-section, better maintaining the pressure and velocity of the blood flow segments and having a smaller impact on local blood flow. The effect is particularly evident when the length-to-width ratio of the elliptical cross-section is greater than 1.5.

Technical personnel in this field can make other modifications within the spirit of the present disclosure. These various modifications derived in accordance with the inventive spirit of the present disclosure should still fall within the scope of protection of the present disclosure.

## Claims

1. An embolic protection device comprising a metal wire or metal ribbon with an elliptical or flat cross-section pre-formed into a spiral shape wound around the short axis, wherein the embolic protection device is configured to be deployed within a tubular delivery system and introduced into a target blood vessel, and the elasticity of the material allows the device to revert to its predetermined shape to intercept emboli.

2. The device of claim 1, wherein the cross-section of the metal wire or metal ribbon is elliptical or streamlined.

3. The device of claim 2, wherein the short axis of the elliptical or streamlined cross-section faces the direction of blood flow and is tangent to it.

4. The device of claim 2, wherein the long axis of the elliptical or streamlined cross-section is parallel to the direction of blood flow.

5. The device of claim 2, wherein the length-to-width ratio of the elliptical or streamlined cross-section is greater than 1.5.

6. The device of claim 2, wherein the ratio of the short axis to the minimum intercept diameter is less than the maximum elastic strain of the material.

7. The device of claim 2, wherein the angle between the axis of the spiral and the long axis of the elliptical or streamlined cross-section is an acute angle.

8. The device of claim 7, wherein the angle is less than 30 degrees.

9. The device of claim 2, wherein the angle between the axis of the spiral and the wide face of the elliptical or streamlined cross-section is an acute angle.
